# EUROPEAN PATENT APPLICATION

(11) **EP 3 081 183 A1**
(43) Date of publication of application: **19.10.2016**
(21) Application number: 14870558.5
(22) Date of filing: 12.12.2014
(51) Int. Cl.: A61B 18/00

(54) **TREATMENT INSTRUMENT AND TREATMENT SYSTEM**

(30) Priority: 13.12.2013 JP 2013258523
(71) Applicant: Olympus Corporation, Tokyo 192-8507 (JP)
(72) Inventor: AKAGANE, Tsunetaka, Hachioji-shi Tokyo 192-8507 (JP); HONDA, Yoshitaka, Hachioji-shi Tokyo 192-8507 (JP); HAYASHIDA, Tsuyoshi, Hachioji-shi Tokyo 192-8507 (JP)
(74) Representative: Schicker, Silvia
(86) International application number: PCT/JP2014/083013
(87) International publication number: WO 2015/088012

(57) **Abstract**

A treatment device includes a support section having conductivity, for which a separate position separated from the probe is switched to a support position at which the support section abuts the probe to support the probe when bent in a direction off of the central axis in accordance with a load which is applied to the probe, and subserviently moved with the vibration of the probe. The support section absorbs the vibration transmitted in the probe when the support section is at the support position.

## Description

### Technical Field

The invention relates to a treatment device and a treatment system which is configured to treat living tissues by ultrasonic vibration.

### Background Art

Jpn. Pat. Appln. KOKAI Publication No. 2012-210445 discloses, for example, a system including a treatment device in which a support section (a contact structure) formed of an electrically conductive material such as stainless steel or titanium, is arranged on an inner circumferential surface of the distal end portion of a cylindrical body (a sheath) and a probe, to which ultrasonic vibration is transmitted, is arranged on the central axis of the cylindrical body. The probe is bent when an external force is applied in a direction orthogonal to the axis direction of the probe, and the probe may contact the support section arranged in the cylindrical body. The system generates an alarm as a trigger at the time of the contact of the probe with the support section. For this reason, the system warns a user that an excessive external force is being applied to the probe, and the user should stop outputting energy such as ultrasonic output, etc. from the treatment device, in order to avoid damage to the probe.

The system disclosed in Jpn. Pat. Appln. KOKAI Publication No. 2012-210445, for example, may issue a warning when the probe contacts the support section during incision of living tissue which is difficult to incise. The system stops outputting energy, such as ultrasonic output, etc., upon detection of a warning. Thus, it may be necessary for the system to temporarily stop the treatment without having finished the incision of living tissue, and to restart the incision of the living tissue.

In the treatment device disclosed in Jpn. Pat. Appln. KOKAI Publication No. 2012-210445, for example, if ultrasonic vibration continues to be transmitted to the probe while the probe is in contact with the support section, the probe may be damaged.

### Summary of Invention

The purpose of the invention is to provide a treatment device and a treatment system that can avoid damaging the probe even when the probe is bent and contact the support section arranged in a cylindrical body, and permits choosing to continue the treatment while the probe is in contact with the support section.

According to one aspect of the present invention, a treatment device includes: a cylindrical body having a central axis; a probe having conductivity in which ultrasonic vibration is transmitted from a proximal end thereof to a distal end thereof and which is inserted into the cylindrical body and extends toward a distal end side with respect to a distal end of the cylindrical body; and a support section having conductivity which is separated forward or backward, along the central axis, of a position corresponding to a node of vibration of the probe in a state when the ultrasonic vibration is transmitted, for which a separate position separated from the probe is switched to a support position at which the support section abuts the probe to support the probe when bent in a direction off of the central axis in accordance with a load which is applied to the probe, and the support section which is subserviently moved with the vibration of the probe and absorbs the vibration transmitted in the probe when the support section is at the support position.

### Advantageous Effects of Invention

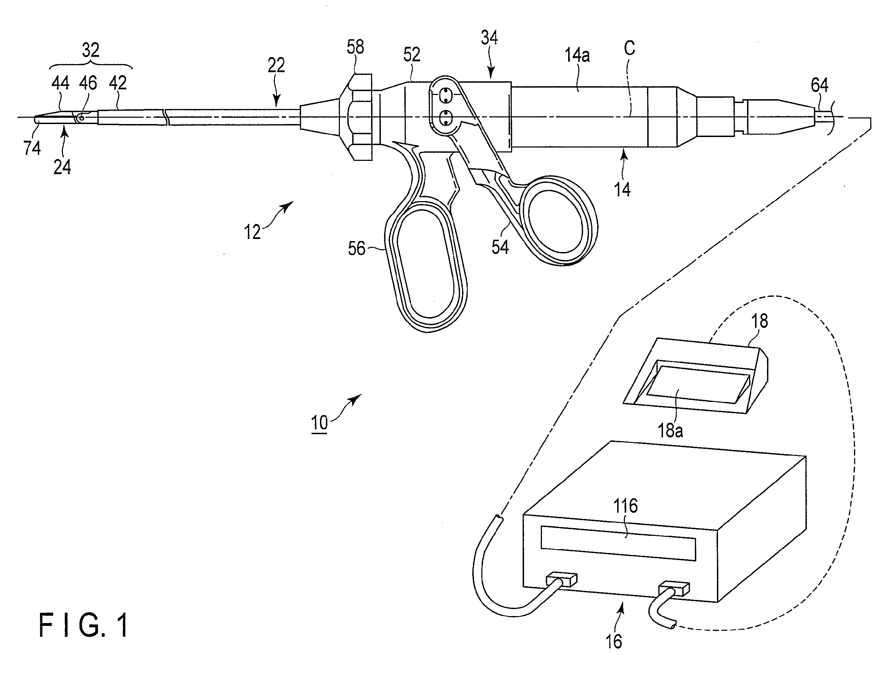

According to the present invention, it is possible to provide a treatment device and a treatment system that can avoid damaging the probe even when the probe is bent and contacts the support section arranged in a cylindrical body, and permits choosing to continue the treatment while the probe is in contact with the support section. Brief Description of Drawings
FIG. 1 is a schematic diagram illustrating a treatment system according to the first embodiment.

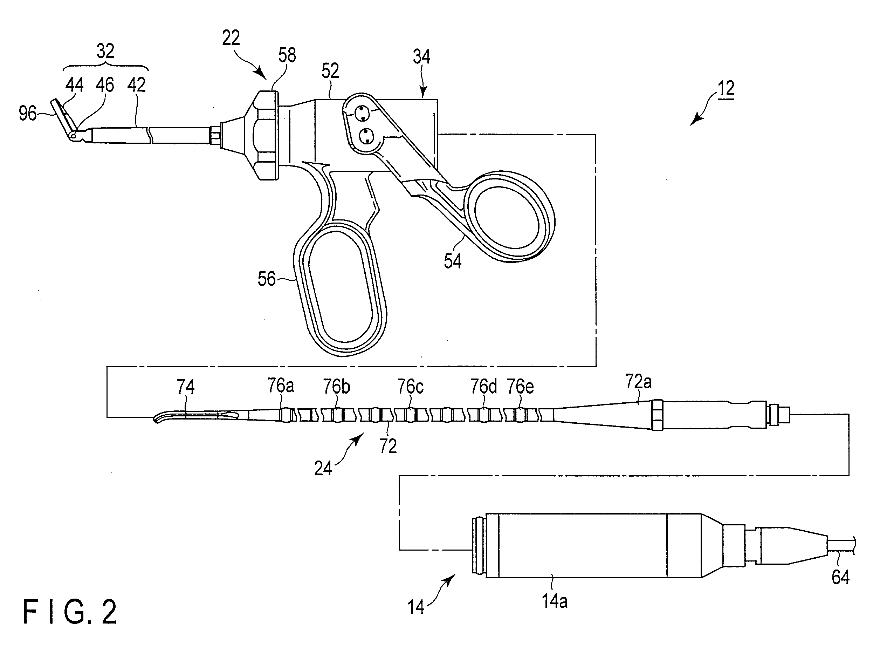

FIG. 2 is a schematic diagram illustrating an exploded view of a treatment unit, a probe, and an ultrasonic transducer unit of the treatment system according to the first embodiment.

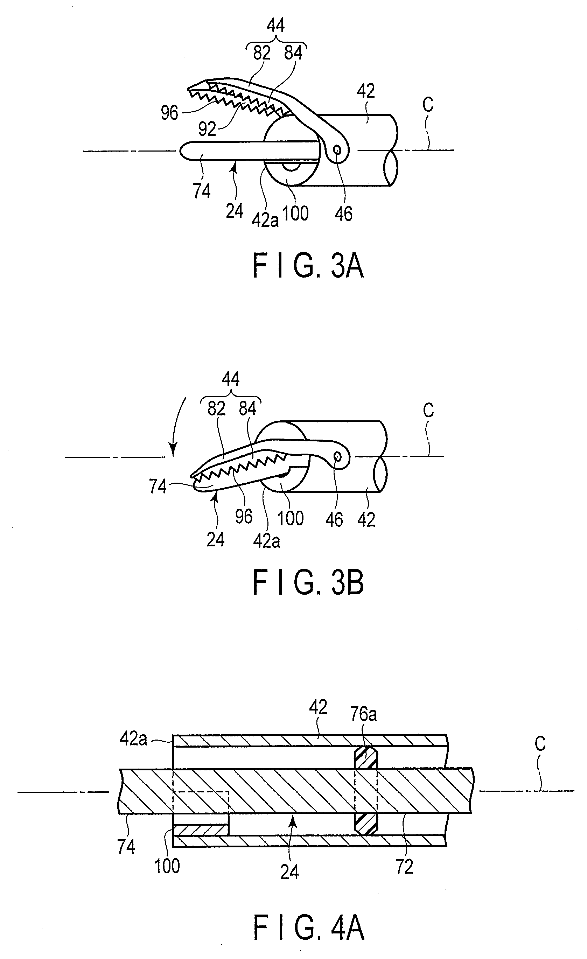

FIG. 3A is a schematic perspective view illustrating the treatment system according to the first embodiment, and illustrating that the action portion is open while being separated from the treatment section of the probe, and the probe is separated from the support section arranged at the distal end of the sheath.
FIG. 3B is a schematic perspective view illustrating the treatment system according to the first embodiment, and illustrating that the action section is closed in the vicinity of the treatment section of the probe, and the probe is supported by being abutted to the support section arranged at the distal end of the sheath.
FIG. 4A is a vertical cross-sectional diagram schematically illustrating the relationship between the support section at the distal end of the sheath and the probe of the treatment system according to the first embodiment, and the state where the support section is arranged in approximately an arc shape on the inner circumferential surface of the distal end portion of the sheath, and the probe is at a separate position separated from the support section.

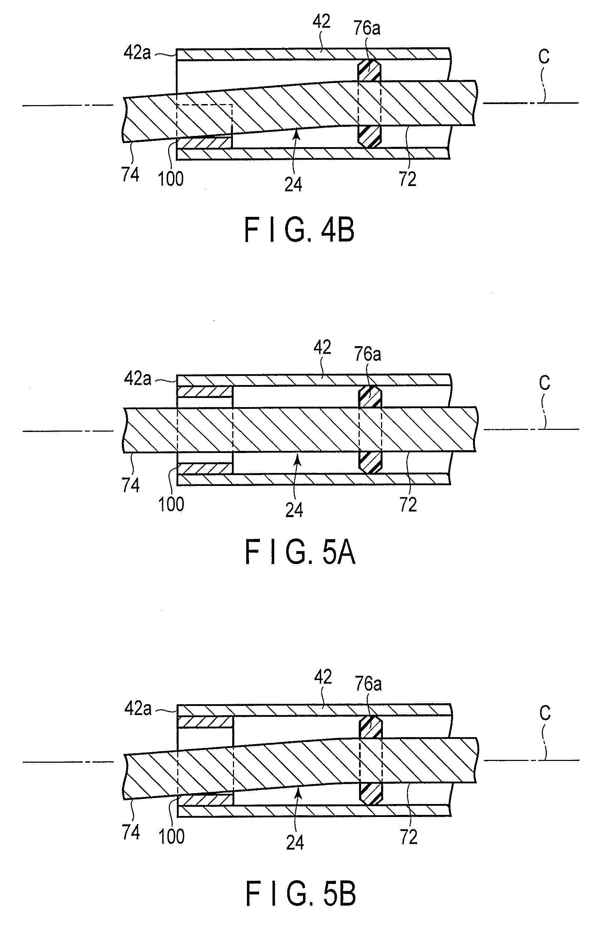

FIG. 4B is a vertical cross-sectional diagram schematically illustrating the relationship between the support section at the distal end of the sheath and the probe of the treatment system according to the first embodiment, and the state where the support section is arranged in approximately an arc shape on the inner circumferential surface of the distal end portion of the sheath, and the probe is at a support position at which the probe is supported by being abutted to the support section.
FIG. 5A is a vertical cross-sectional diagram schematically illustrating the relationship between the support section at the distal end of the sheath and the probe of the treatment system according to the first embodiment, and the state where the support section is arranged in a ring shape on the inner circumferential surface of the distal end portion of the sheath and the probe is at a separate position separated from the support section.
FIG. 5B is a vertical cross-sectional diagram schematically illustrating the relationship between the support section at the distal end of the sheath and the probe of the treatment system according to the first embodiment, and the state where the support section is arranged in a ring shape on the inner circumferential surface of the distal end portion of the sheath and the probe is at a support position at which the probe is supported by being abutted to the support section.

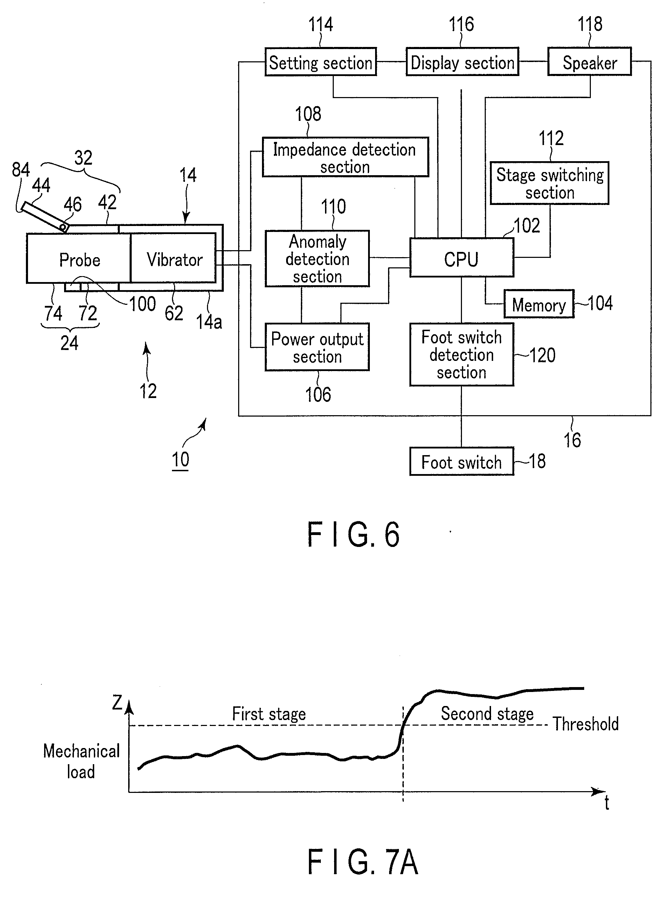

FIG. 6 is a block diagram schematically illustrating a treatment system according to the first embodiment.
FIG. 7A is a schematic diagram illustrating an example of a change in mechanical loads to the probe (difficulty of probe movement) by measuring acoustic impedance of a transducer.

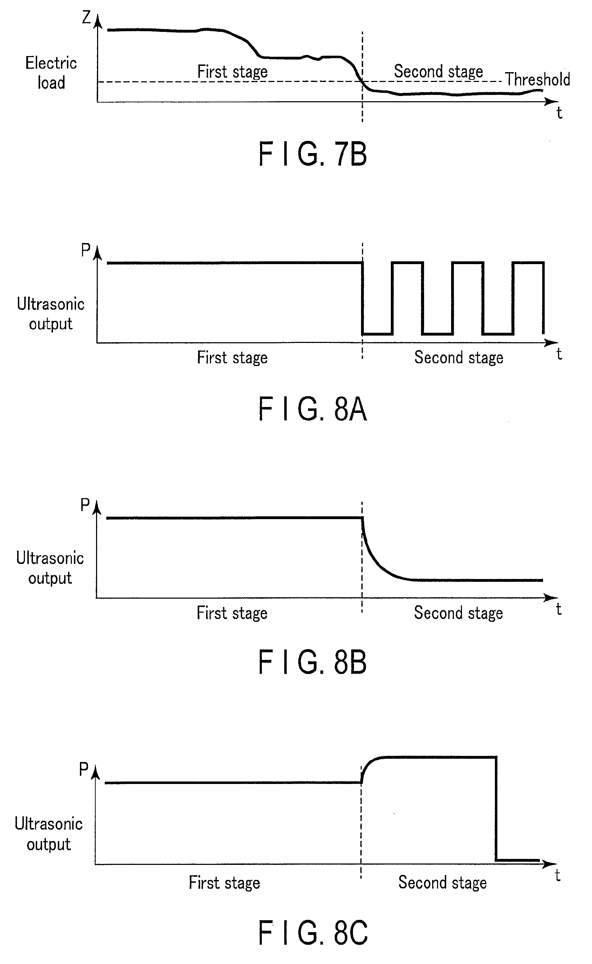

FIG. 7B is a schematic diagram illustrating an example of a change in an electric load between the probe and the support section (when the probe is in the vicinity of the support section) obtained by measuring the ease of the flow of electricity between the probe and the support section.
FIG. 8A is a schematic diagram illustrating an example of the first stage in which the amplitude of the ultrasonic vibration is kept constant at the separate position where the probe is separated from the support section, and the second stage in which the amplitude of the ultrasonic vibration is changed at the support position at which the probe is supported by being abutted to the support section.
FIG. 8B is a schematic diagram illustrating an example of the first stage in which the amplitude of the ultrasonic vibration is kept constant at the separate position where the probe is separated from the support section, and the second stage in which the amplitude of the ultrasonic vibration is changed at the support position at which the probe is supported by being abutted to the support section.
FIG. 8C is a schematic diagram illustrating an example of the first stage in which the amplitude of the ultrasonic vibration is kept constant at the separate position where the probe is separated from the support section, and the second stage in which the amplitude of the ultrasonic vibration is changed at the support position at which the probe is supported by being abutted to the support section.

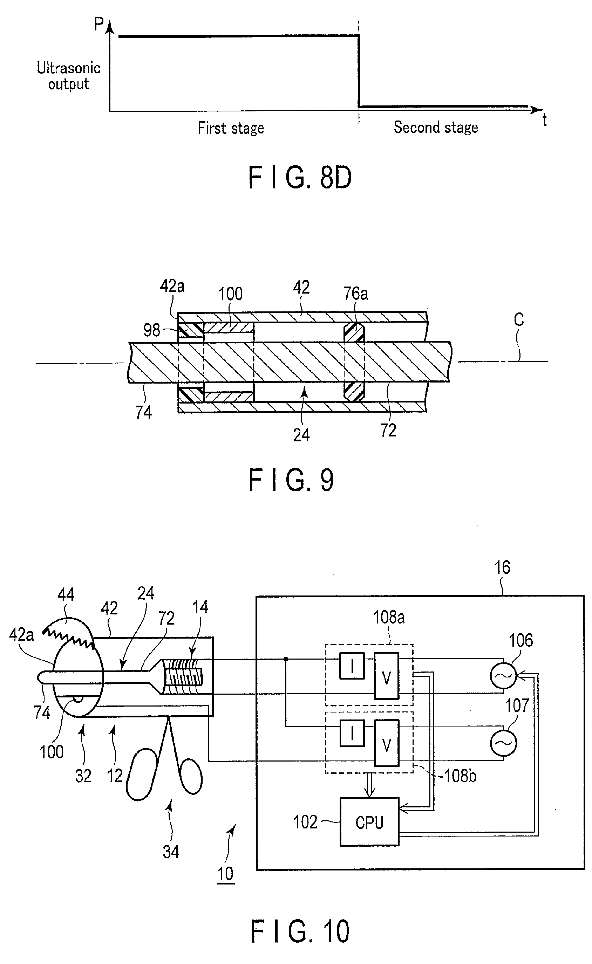

FIG. 8D is a schematic diagram illustrating an example of the first stage in which the amplitude of the ultrasonic vibration is kept constant at the separate position where the probe is separated from the support section, and the second stage in which the amplitude of the ultrasonic vibration is changed at the support position at which the probe is supported by being abutted to the support section.
FIG. 9 is a vertical cross-sectional diagram schematically illustrating the relationship among a resin material of the distal end portion of the sheath, the support section, and the probe of the treatment system according to the first variation of the first embodiment, and the state where the resin material and the support section are arranged in a ring shape on the inner circumferential surface of the distal end portion of the sheath, and the probe is at a separate position separated from the resin material and the support section.
FIG. 10 is a schematic diagram illustrating a treatment system according to the second variation of the first embodiment.

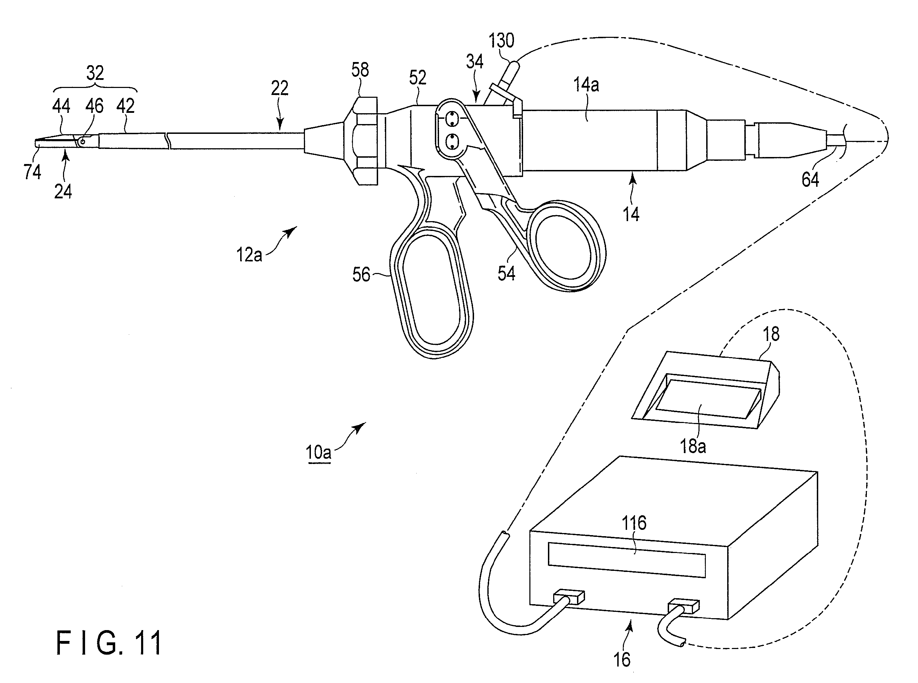

FIG. 11 is a schematic diagram illustrating a treatment system according to the second embodiment.

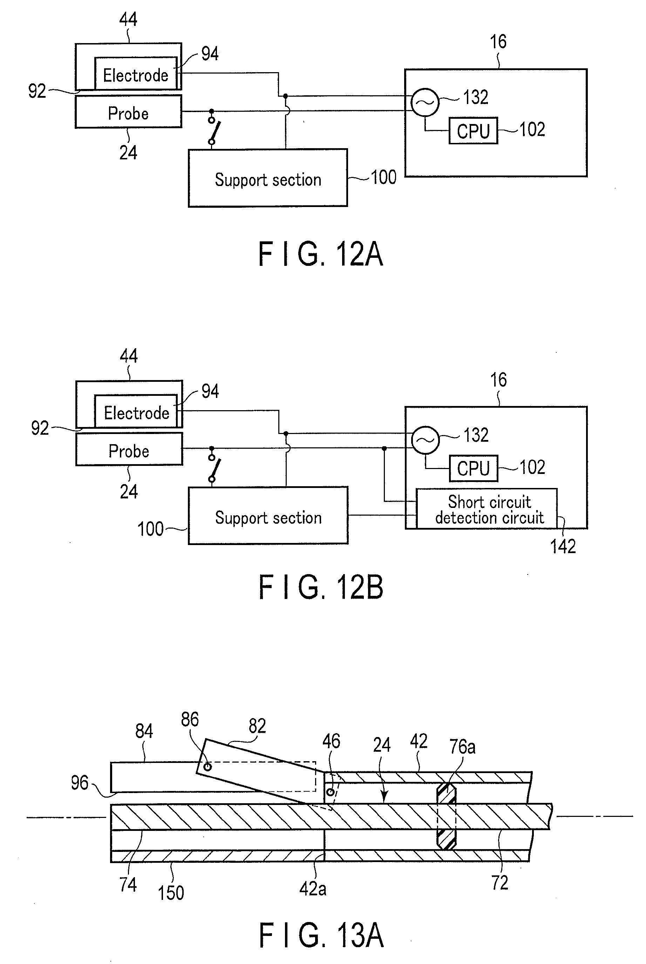

FIG. 12A is a schematic diagram illustrating the relationship among an action section having electrodes, a probe, a support section and a controller of the treatment system according to the second embodiment.
FIG. 12B is a schematic diagram illustrating the relationship among an action section having electrodes, a probe, a support section and a controller having a short circuit detection circuit of the treatment system according to a variation of the second embodiment.
FIG. 13A is a vertical cross-sectional diagram schematically illustrating the relationship between the probe cover as a support section extending from the distal end of the sheath toward the distal end direction and the probe of the treatment system according to the third embodiment, and the state where the probe cover is arranged in approximately an arc shape (a half-pipe shape) with respect to the distal end portion of the sheath on the distal end side, and the probe is at a separate position separated from the probe cover.

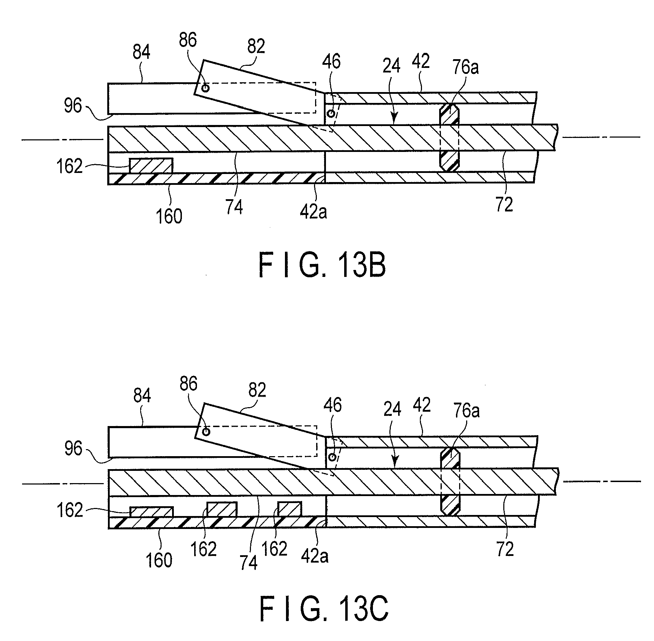

FIG. 13B is a vertical cross-sectional diagram schematically illustrating the relationship between the probe and the support section which is arranged on the probe cover having electrical insulation properties and extending from the distal end of the sheath toward the distal end direction of the treatment system according to the first variation of the third embodiment, and the state where only one support section is arranged in the distal end portion of the sheath on the distal end side, and the probe is at a separate position separated from the support section.
FIG. 13C is a vertical cross-sectional diagram schematically illustrating the relationship between the probe and the support section which is arranged on the probe cover having electrical insulation properties and extending from the distal end of the sheath toward the distal end direction of the treatment system according to the first variation of the third embodiment, and the state where a plurality of support sections are arranged in the distal end portion of the sheath on the distal end side, and the probe is at a separate position separated from the support section.

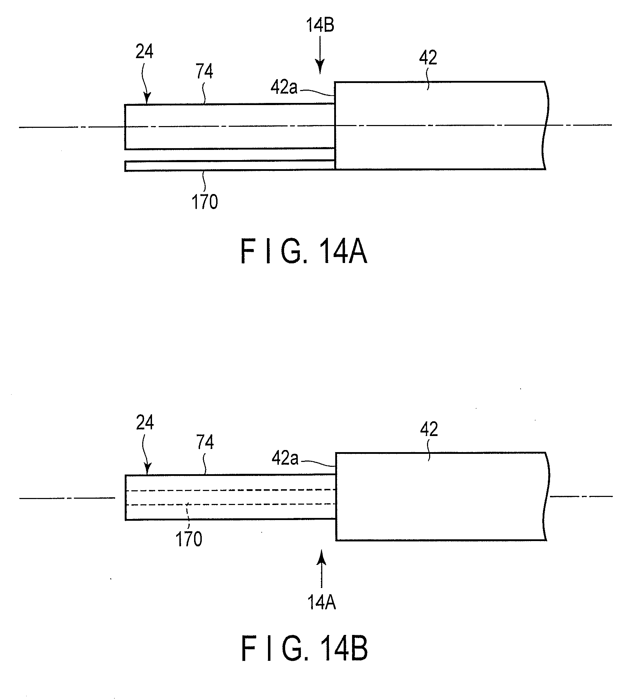

FIG. 14A is a schematic side view from the direction of arrow 14A shown in FIG. 14B, illustrating the relationship between the probe and the extended portion as a support section extending from the distal end of the sheath toward the distal end direction of a treatment system according to the second variation of the third embodiment, and the state where the width of the extended portion is smaller than the diameter of the treatment section of the probe, and the probe is at a separate position separated from the support portion.
FIG. 14B is a schematic top view from the direction of arrow 14B shown in FIG. 14A, illustrating the relationship between the probe and the extended portion as a support section extending from the distal end of the sheath toward the distal end direction of a treatment system according to the second variation of the third embodiment, and the state where the width of the extended portion is smaller than the diameter of the treatment section of the probe, and the probe is at a separate position separated from the support section.

### Brief Description of Embodiments

Hereinafter, embodiments of the present invention will be described with reference to the drawings.

The first embodiment will be explained with reference to FIG. 1 to FIG. 8D.

As shown in FIG. 1, the treatment system 10 according to the first embodiment includes a treatment device 12, a transducer unit 14, a controller 16 used as a power source, and a foot switch 18.

As shown in FIGS. 1 and 2, the treatment device 12 includes a treatment unit 22 and the probe 24, which can be mutually assembled and disassembled. The probe 24 is inserted into the sheath 42 of the treatment unit 22 which will be described later. The transducer unit 14 is detachably connected to the treatment device 12. Specifically, the proximal end portion of the probe 24 is connected to the transducer unit 14 in the inside of the treatment unit 22. Thus, ultrasonic vibration is transmitted to the probe 24 from its proximal end to its distal end.

The treatment device 12 is held by a user, and is capable of cutting living tissue using ultrasonic vibration generated at a transducer 62 (described later) of the transducer unit 14. The treatment device 22 has an insertion section 32 and an operation section 34. The insertion section 32 includes a sheath 42 as a thin cylindrical body having a central axis C, and an action portion (a moving body) 44. The sheath 42 is formed of a metallic material, such as stainless alloy, etc. The outer circumferential surface of the sheath 42 is covered with a resin material having electrical insulation properties. The action portion 44 is arranged at the distal end of the sheath 42, and the proximal end of the action portion 44 is rotatable by the pivotal shaft 46. The action portion 44 and the probe 24, which is arranged along the axis direction of the sheath 42, are arranged side-by-side in line. The action portion 44 can come close to and away from the treatment section 74 (described later) of the probe 24, which projects toward the distal end side with respect to the distal end 42a of the sheath 42; in other words, the action portion 44 is adjustable to open and close with respect to the treatment section 74. Thus, the action portion 44 can apply an external force to the treatment section 74 of the probe 24 in a direction off of the central axis C. The probe 24 is bent when a load (external force) is applied to the treatment section 74 in a direction off of the central axis C, and the probe 24 returns to its original state (straight) when the load is removed.

The action portion 44 and the treatment section 74 of the probe 24 constitute a holding section that holds living tissue.

The operation section 34 is arranged at the proximal end of the sheath (cylindrical body) 42. The operation section 34 includes an operation main body 52 and a movable handle 54 which makes the action portion 44 close to or separated from the treatment section 74 of the probe 24. The operation main body 52 is formed in a cylindrical shape, and the transducer unit 14 is detachably attached to the proximal end portion of the operation main body 52. The central axis of the probe 24, the central axis of the ultrasonic oscillator (ultrasonic transducer) 62 of the transducer unit 14, the central axis of the operation main body 52, and the central axis of the sheath 42 are the same axis.

The operation main body 52 has a stationary handle 56. The stationary handle 56 extends toward the radial direction of the cylindrical operation main body 52. The movable handle 54 is supported by the operation main body 52 so as to be arranged side by side in line with the stationary handle 56. In the present embodiment, the movable handle 54 is arranged behind the fixed handle 56; however, the moving handle 54 may be arranged in front of the stationary handle 56. The movable handle 54 can be brought close to or separated from the stationary handle 54 by a known mechanism. The action section 44 can be rotated with respect to the distal end of the sheath 42.

The operation section 34 has a rotating knob 58. The rotating knob 58 is located in front of the operation main body 52, and is capable of rotating the sheath 42 and the action section 44 about the axis with respect to the probe 24. In other words, when the rotating knob 58 is turned around the axis with respect to the action portion 34, the sheath 42 and the action section 44 can be integrally rotated about the axis with respect to the vibration transmitting section 72 of the probe 24. Thus, the action portion 44 pivoted at the distal end portion of the sheath 42 rotates about the axis with respect to the probe 24.

The transducer unit 14 is detachably arranged at the proximal end portion of the operation main body 52. Preferably a BLT-type ultrasonic vibrator (ultrasonic transducer) 62 (see FIG. 6) which generates ultrasonic vibration, for example, is stored inside of the transducer unit 14 (inside of the cover 14a). A cable 64 extends from the proximal end portion of the transducer unit 14. An end of the cable 64 is connected to the controller 16 shown in FIG. 1. In other words, the ultrasonic transducer 62 of the ultrasonic transducer unit 14 is connected to the controller 16 to supply power to the ultrasonic transducer 62. When the ultrasonic transducer 62 is supplied with necessary power from a power output section 106 (described later) of the controller 16, the ultrasonic transducer 62 is driven to generate ultrasonic vibration. In other words, the ultrasonic transducer 62 of the ultrasonic transducer unit 14 is arranged at the proximal end of the probe 24, and can oscillate ultrasonic vibration from the proximal end to the distal end of the probe 24, upon the power supply from the controller 16. Thus, the vibration generated at the ultrasonic transducer 62 can be transmitted to the probe 24.

The probe 24 shown in FIG. 2 is made of a metallic material such as titanium alloy, for example, and is formed in the shape of rod. The probe 24 includes a thin vibration transmitting section 72 having a horn 72a to amplify an amplitude, and the treatment section 74 which is formed integrally with the vibration transmitting section 72 on the distal end side. The probe 24 and the transducer unit 14 are connected by screwing the proximal end of the vibration transmitting section 72 into the distal end of the transducer unit 14. The vibration transmitting section 72 transmits ultrasonic vibration generated at the ultrasonic transducer 62 of the transducer unit 14 from the proximal end to the distal end. The vibration transmitting unit 72 transmits ultrasonic vibration to the treatment unit 74 which is arranged at the distal end side of the vibration transmitting unit 72.

The length of the probe 24 is determined by a resonance frequency of the ultrasonic transducer 62 of the ultrasonic transducer unit 14. For example, if the resonance frequency of the longitudinal vibration at the ultrasonic transducer 62 is 47kHz, one wavelength is approximately 100 mm, and thus, the node position of the vibration at the probe 24 is at the intervals of approximately 50 mm. The treatment section 74 of the probe 24 is located at and near the anti-node position of the vibration. The resonance frequency of the ultrasonic transducer 62 is not limited to 47kHz, and it may be 23.5kHz, etc.

As shown in FIG. 2, a plurality of holding members 76a, 76b, etc. are provided on the outer circumference of the vibration transmitting unit 72, and the holding members are spaced in the axis direction. For example, the holding member 76a is arranged at a position which is a node position of the ultrasonic vibration of the vibration transmitting section 72, and is the closest to the distal end. In a case where the resonance frequency of the ultrasonic transducer 62 is 47kHz, the holding member 76b is arranged at a location in a distance of approximately 50 mm from the holding member 76a on the proximal end side, and the holding members 76c, 76d, etc. are arranged at specified positions after the holding member 76b at the intervals of approximately 50 mm. Each of the holding members 76a, 76b, etc. is made of ring-shaped rubber material having non-conductivity (electrical insulation properties). The vibration transmitting section 72 on which the holding members 76a, 76b, etc. are arranged is inserted in the sheath 42. The holding members 76a, 76b, etc. prevent contact of the vibration transmitting section 72 with the inner circumferential surface of the sheath 42, which is made of a metallic material.

Herein, as shown in FIG. 1, the treatment section 74 of the probe 24 projects toward the distal end of the sheath 42 on the distal end side. Thus, the action portion 44 faces the treatment section 74 in such a manner that the action portion 44 can be close to or separated from the treatment section 74 of the probe 24.

As shown in FIGS. 3A and 3B, the action portion 44 comprises a jaw 82 which is operated by the movable handle 54 and a pressing section 84 which is arranged in the jaw 82 and faces the treatment section 74 of the probe 24 to press down and grip living tissue. The pressing section 84 has a pad (a shock absorbing section) 92 made of a resin material such as PTFE having heat resistance, abrasion resistance, and non-conductivity properties (electrical insulation properties). The pad 92 forms a gripping surface 96 to grip living tissue. The surface of the pad 92 facing the treatment section 74 (i.e., the gripping surface 96) is preferably formed as a non-slip surface with serrations or a satin finish, for example.

As shown in FIGS. 3A to 5B, in the present embodiment, a support section (a probe retainer) 100 which is made of a metallic material such as a vibration-dampening alloy and acts, for example, as a vibration dampener, is arranged on the inner circumferential surface of or near the distal end 24a of the sheath 42. The support section 100 is formed in approximately an arc shape with a certain angle (approximately U-shaped), or in a ring shape and has abrasion resistance and conductivity. The support section 100 prevents abrasion of the probe 24 caused by ultrasonic vibration transmitted to the probe 24, and preferably has abrasion resistance to prevent abrasion to the support section 100 itself by the probe 24. The support section 100 is arranged opposite to the action portion 44 with respect to the probe 24. Note that the action portion 44 is omitted in FIGS. 4A to 5B.

The inner circumferential surface of or near the distal end portion 42a of the sheath 42 at which the support section 100 is arranged is located ahead of or behind, along the central axis C, a position corresponding to a node of the vibration of the probe 24 at a state when ultrasonic vibration is transmitted. In the present embodiment, for example, the support section 100 is preferably located along the central axis C at a position ahead of and separated from the position corresponding to the node of the vibration of the probe 24 in a state when ultrasonic vibration is transmitted (the position where the holding member 76a is arranged).

As shown in FIGS. 3A to 4B, when the support section 100 has an approximate arc shape with a certain angle, it is possible to support the probe 24 which is pressed down by the action portion 44 by abutting the probe 24 to the support section 100.

As shown in FIGS. 5A and 5B, when the support section 100 has a ring shape, the sheath is preferably open on its central axis so that the proximal end of the probe 24 can be inserted into the proximal end side of the sheath 42 through the distal end of the sheath 42 and the support section 100. Furthermore, the support section 100 in this case can support the probe 24 pressed down by the action portion 44 by abutting the probe 24 to the support section 100 itself, and can also support the probe 24 by abutting the probe 24 to the support section 100 when a load (external force) is applied to the treatment section 74 of the probe 24 from the side opposite of the action portion 44, for example.

Thus, when the probe 24 is bent by a load applied to the treatment section 74, etc. of the probe 24 in a state when ultrasonic vibration is transmitted, the probe 24 may be abutted to the support section 100. The support section 100 therefore can support the bent probe 24. Therefore, the support section 100 defines a maximum bend allowance of the probe 24 at or near the distal end of the sheath 42.

The support section 100 is made of a material that prevents abrasion of the probe 24 in a state when the probe 24 is abutted to the support section 100 and ultrasonic vibration is transmitted to the probe 24, and prevents abrasion to the support section 100 itself by the probe 24 in the state when ultrasonic vibration is transmitted. In other words, it is possible to prevent damaging the probe 24 by the support section 100 abutting against the probe 24 in a state when ultrasonic vibration is transmitted, and to prevent damaging the support section 100 by the probe 24.

The support section 100 is made of a material that is interlocked and moved together with the vibration of the probe 24 when the probe 24 is bent to abut against the support section 100 while ultrasonic vibration is transmitted to the probe 24. In other words, the support section 100 is resonant with the vibration of the probe 24 in a state when ultrasonic vibration is transmitted. Furthermore, the support section 100 is made of a material that absorbs vibration transmitted to the probe 24 when the probe 24 is bent to abut against the support section 100. At this time, the support section 100 generates heat by absorbing vibration transmitted to the probe 24.

As a material for the support section (vibration dampener) 100, a vibration-dampening alloy, for example, is preferable. For example, an alloy of iron and aluminum (an Al-Fe alloy) having a maximum loss factor of 0.07, for example, and a dampening capacity of 10% or more is used. If a vibration-dampening alloy is composed of Al-Fe, the Al content is preferably 6 to 10 weight percent, more preferably 8 weight percent. If the support section 100 made of the Al-Fe alloy with the Al content of 8 weight percent is used, the support section 100 has a higher heat resistance and a higher abrasion resistance than the pad 92 made of a PTFE material.

The vibration-dampening alloy used as the material for the support section 100 has a high rigidity; thus, it is capable of absorbing vibration even when an amount of deformation is small. As a vibration-dampening alloy, there are alloys made by dislocation, twinning deformation, composite structure, internal friction, or a combination of any of the aforementioned. If an alloy is made by dislocation, for example, it is possible to absorb vibration energy by producing an energy loss (energy loss caused by the dislocation) caused by the interaction between the dislocation and impurity in the crystal. If an alloy is made by twinning deformation, it is possible to absorb vibration energy by producing twinning deformation in the vibration-dampening alloy.

A vibration-dampening alloy preferably has the same or higher strength as iron, i.e., has an excellent specific strength, such as a material having a modulus of elasticity at the same level as iron, while also having a weight approximately 10% lighter than iron. A vibration-dampening alloy is preferably easy to forge, stamp, and cut. Furthermore, a vibration-dampening alloy preferably has anti-oxidation properties due to an oxidation layer that is stable in both low and high temperatures, and does not easily fracture at room temperature due to brittleness, and can be easily processed into complicated shapes. While a vibration-dampening alloy is metal and has conductivity, the resistance value thereof is, for example, preferably four times or approximately a few times larger than that of iron.

For example, other than an Al-Fe alloy, a composite-type Al-Zn alloy, a twinned Ni-Ti alloy, Cu-Al-Ni alloy, Mn-Cu alloy, Mn-Cu-Ni-Fe alloy, etc. can be used as appropriate as a vibration-dampening alloy. If a vibration-dampening alloy is used for the support section 100 according to the present embodiment, the dampening capacity of the alloy is preferably 10% or larger.

In the present embodiment, the probe 24 to which ultrasonic vibration is transmitted is switched between the separate position separated from the support section 100 (see FIGS. 3A, 4A, and 5A), and the support position at which the probe 24 is supported by being abutted to the support section 100 (see FIGS. 3B, 4B, and 5B).

Herein, when the probe 24 is at the separate position in relation to the support section 100, the controller 16 performs a constant current control when driving the ultrasonic transducer 62 to maintain the amplitude of the treatment section 74 of the probe 24 by the ultrasonic vibration. Such control at the separate position by the controller 16 is called a first stage control.

The control at the support position by the controller 16 after switching from the separate position to the support position is called a second stage control. In the second stage, the stage switching unit 112 (described later) of the controller 16 performs a control set as appropriate by the setting unit 114 in accordance with the user's preference and a treatment target to proceed with the treatment of living tissue or stop the treatment temporarily. In other words, the controller 16 in the second stage may perform a constant current control or a control to vary a current as appropriate. It should be noted that the control by the controller 16 is continuous when switching the control from the first stage to the second stage.

As shown in FIG. 6, the controller 16 includes a CPU 102, a memory 104, a power output section (AC power source section) 106, an impedance detection section (detection section) 108, an anomaly detection section 110, a stage switching section 112, a setting section 114, a display section 116, a speaker (alarm generating section) 118, and a foot switch detection section 120. The memory 104, the power output section 106, the impedance detection section (detection section) 108, the anomaly detection section 110, the stage switching section 112, the setting section 114, the display section 116, the speaker (alarm generating section) 118, and the foot switch detection section 120 are connected to the CPU 102 to transmit and receive electric signals. The foot switch detection section 120 detects an operation of a pedal 18a of the foot switch 18.

The memory 104 stores, based on a value determined by the setting section 114, a maximum voltage, etc. and a threshold (see FIG. 7A), etc. of a mechanical load (difficulty of probe 24 movement) for the probe 24 which is detected by the impedance detection section 108.

The power output section 106 and the impedance detection section 108 are connected to the transducer 62 of the transducer unit 14. The anomaly detection section 110 is connected to the power output section 106 and the impedance detection section 108. The anomaly detection section 110 can detect anomalies in the amount of output at the power output unit 106, and anomalies in a value detected by the impedance detection section 108, and the like.

The controller 16 is configured to not output power to the transducer 62 when an anomaly is detected by the anomaly detection section 110, for example, when the probe 24 is not connected at the time of outputting power from the power output section 106 to the transducer 62, or when a short circuit occurs in the probe 24.

FIG. 7A shows that a mechanical load to the probe 24 from the beginning to the end of a treatment is obtained by measuring acoustic impedance of the transducer 62 at the impedance detection section 108. When the probe 24 is abutted to the support section 100, the bend of the probe 24 is limited by the support section 100; thus, a mechanical load, i.e., acoustic impedance of the transducer 62, rises rapidly. In a state when ultrasonic vibration is transmitted, when the probe 24 is abutted to the support section 100 and is switched from the separate position to the support position, the impedance detection section 108 can detect the switch. If a threshold is empirically set for acoustic impedance of the transducer 62 at this time, it is possible to recognize switching of the probe 24 from the separate position to the support position with respect to the support section 100 (an anomaly in a value detected by the impedance detection section 108).

The threshold is shown as a fixed value in FIG. 7A; however, the threshold may be determined by an appropriate function based on an initial value, etc. of a load at the beginning of a treatment. In this case, the threshold changes in accordance with the initial value, etc. of the load. It may also be preferable to determine that the probe 24 is switched from the separate position to the support position based on the amount of change per unit of time. This is the same for the threshold of an electric load (see FIG. 7B), which will be described later.

Next, the action of the treatment system 10 according to the present embodiment is described.

The user attaches the treatment unit 22 with the probe 24 to form the treatment device 12. At this time, the support section 100 on the inner circumferential surface of or near the distal end portion 42a of the sheath 42 is at a separate position separated from the probe 24. The user further attaches the treatment unit 22 and the probe 24 to the transducer unit 14. Then, the user connects the transducer unit 14 to the controller 16. The user sets treatment and control details for the treatment as appropriate with the setting section 114.

The user operates the movable handle 54 of the operation section 34 to bring the action portion 44, which was previously separated from the treatment section 74 of the probe 24, close to the treatment section 74, and grips living tissue between the treatment section 74 of the probe 24 and the gripping surface 96 of the pressing section 84. In other words, the user presses down living tissue with the pressing section 84 against the treatment section 74 of the probe 24. For this reason, the probe 24 is bent when the living tissue is held by the pressing pressure of the pressing section 84 (a load in a direction off of the axis direction with respect to the central axis C). Thus, the probe 24 is bent in a direction off of the central axis C in accordance with the load. Furthermore, the probe 24 is brought close to the support section 100 which is arranged on or near the inner circumferential surface of the distal end portion 42a of the sheath 42. Suppose the probe 24 is at the separate position separated from the support section 100 at this time.

In this state, the user presses down the pedal 18a of the foot switch 18 to output power from the power output section 106 to the transducer 62 of the transducer unit 14 to generate ultrasonic vibration at the transducer 62. At this time, the probe 24 is at the separate position separated from the support section 100, and the controller 16 therefore performs a first stage control.

The ultrasonic vibration generated by the transducer 62 is transmitted to the treatment section 74 through the vibration transmitting section 72 of the probe 24. Frictional heat is generated between the treatment section 74 of the probe 24 to which ultrasonic vibration is transmitted and living tissue pressed down by the action portion 44 toward the treatment section 74, thereby clotting the living tissue to provide hemostasis and incising the living tissue. Thus, it is possible to cut the living tissue pressed down toward the treatment section 74 into pieces with the treatment section 74 of the probe 24.

At this time, as an example, the mechanical load against the probe 24 changes as shown in FIG. 7A, and the electric load between the probe 24 and the support section 100 changes as shown in FIG. 7B. In the present embodiment, the controller 16 performs constant current control during the first stage to maintain a constant amplitude of the treatment section 74 of the probe 24. Thus, as shown in FIGS. 8A to 8D, the amplitude of the treatment section 74 of the probe 24 is maintained at a constant level. If the amplitude of the treatment section 74 is maintained at a constant level, a voltage is varied in accordance with the fluctuations in the impedance Z shown in FIG. 7A.

Since the living tissue is pressed down by the pressing section 84 toward the treatment section 74 of the probe 24, the probe 24 in a state when vibration is transmitted is bent toward the support section 100. If the living tissue that is pressed down toward the treatment section 74 of the probe 24 in a state when vibration is transmitted is relatively thick or difficult to cut, the user brings the action portion 44 close to the treatment section 74 of the probe 24 to increase the pressing pressure to press down the living tissue toward the treatment section 74 of the probe 24. Thus, the probe 24 is further bent by an excessive tension applied to the treatment section 74 of the probe 24. At this time, the probe 24 may be abutted against the support section 100. The support section 100 can support the bent probe 24, and the probe 24 is switched from the separate position at which the probe 24 is separated from the support section 100 to the support position at which the probe is supported by being abutted to the support section 100.

As ultrasonic vibration from the ultrasonic transducer unit 14 is being input to the proximal end of the probe 24, if the probe 24 contacts the support section 100 made of a vibration-dampening alloy, the support section 100 is subserviently moved with (resonates with) the vibration of the probe 24. Thus, the support section 100 made of a vibration-dampening alloy is moved depending on the vibration of the treatment section 74 so as to vibrate the support section together with the treatment section 74. Accordingly, the support section 100 made of a vibration-dampening alloy appears as if it is attached to the probe 24. Since the support section 100 made of a vibration-dampening alloy is moved together with the vibration of the probe 24, it is possible to prevent damaging the probe 24 by the support section 100 when the support section 100 is abutted to the probe 24 in a state when ultrasonic vibration is transmitted, thereby preventing breakage of the probe 24. Furthermore, when the probe 24 is abutted to the support section 100, the support section 100 has abrasion resistance properties; thus, it is possible to prevent damaging or breaking the support section 100. Since the support section 100 made of a vibration dampening alloy synchronizes the vibration of the probe 24, the support section 100 absorbs vibration energy transmitted to the probe 24; therefore there is an energy loss while the ultrasonic transducer vibrates.

Thus, the vibration energy is transmitted from the probe 24 and is absorbed by the supported section 100 made of a vibration dampening alloy, thereby exercising a braking effect to the ultrasonic vibration of the probe 24.

When the probe 24 is bent, the probe 24 is supported at the support position by being abutted to the support section 100, which is subserviently moved together with the probe 24 and absorbs the vibration. Thus, it is possible to prevent ultrasonic vibration transmitted to the probe 24 from being transmitted to the sheath 24 through the support section 100 when the probe 24 is bent. In other words, it is possible to prevent the ultrasonic vibration transmitted to the probe 24 from affecting the sheath 42 through the support section 100 when the probe 24 is bent. Thus, it is possible to prevent not only breakage of the probe 24 and the support section 100, but also breakage of the sheath 42 when the probe 24 to which ultrasonic vibration is transmitted is abutted to the support section 100.

Furthermore, when the probe 24 contacts the support section 100 while ultrasonic vibration is input to the probe 24, a mechanical load to the probe 24 rapidly rises, as shown in FIG. 7A. Furthermore, when the impedance Z shown in FIG. 7A reaches a threshold (when the probe 24 is switched from the separate position to the support position), the stage switching section 112 of the controller 16 switches the control from the first stage control to the second stage control.

When the probe 24 to which vibration is transmitted is abutted to the support section 100, the impedance detection section 108 and the anomaly detection section 110 of the controller 16 determine that the probe 24 is switched from the separate position to the support position. Upon this determination, the controller issues an alarm sound from the speaker (alarm sound generation section) 118, or displays the switch from the separate position to the support position on the display section 116. These alarms and displays are used as a sign of completion of a series of treatments using the treatment system 10 (see FIG. 8D) or completion in a short time (see FIGS. 8A to 8C).

The stage switching section 112 of the controller 16 switches the control to the second stage control. Herein, the setting section 114 shown in FIG. 6 sets the control in such a manner that ultrasonic vibration is output intermittently or continuously, as shown in FIGS. 8A to 8C. In these cases, it is possible to transmit ultrasonic vibration to the probe 24 as needed after switching from the separate position to the support position.

In the example shown in FIG. 8A, when the probe 24 in the first stage that is abutted to the support section 100 is switched to the support position (second stage), the controller 16 supplies power to the ultrasonic transducer 62 so as to make the amplitude smaller at the beginning of the second stage compared to the amplitude at the time immediately before the impedance detection section 108 detects switching. After that, the controller 16 outputs ultrasonic vibration intermittently, maintaining the state where the probe 24 is supported by the support section 100. Thus, by outputting ultrasonic vibration intermittently, it is possible to prevent heat generation at the probe 24, and heat generation and transmission at the support section 100, while continuing the incision of living tissue. In FIG. 8A, the maximum amplitude of the ultrasonic vibration at the second stage is depicted as the same as that in the first stage; however, the amplitude may be made larger or smaller. The controller 16 completely stops ultrasonic vibration when a certain length of time elapses after the probe 24 is switched from the separate position to the support position.

In the example shown in FIG. 8B, when the probe 24 in the first stage of being abutted to the support section 100 is switched to the support position (second stage), the controller 16 supplies power to the ultrasonic transducer 62 so as to make the amplitude gradually smaller at the beginning of the second stage compared to the amplitude at the time immediately before the impedance detection section 108 detects switching. After that, the controller 16 outputs ultrasonic vibration continuously, maintaining the state where the probe 24 is supported by the support section 100; however, the controller makes the amplitude smaller than that at the first stage and continues outputting the ultrasonic vibration at a constant level. Thus, since the ultrasonic vibration is continued with a decreased amplitude, it is possible to continue the incision of living tissue. The controller 16 completely stops ultrasonic vibration when a certain length of time elapses after the probe 24 is switched from the separate position to the support position.

In the example shown in FIG. 8C, when the probe 24 in the first stage of being abutted to the support section 100 is switched to the support position (second stage), the controller 16 supplies power to the ultrasonic transducer 62 so as to make the amplitude larger at the second stage compared to the amplitude at the time immediately before the impedance detection section 108 detects switching. After that, the controller 16 outputs ultrasonic vibration continuously, maintaining the state where the probe 24 is supported by the support section 100, makes the amplitude smaller than that at the first stage, and continues outputting the ultrasonic vibration at a constant level. The controller 16 completely stops ultrasonic vibration when a certain length of time elapses after the probe 24 is switched from the separate position to the support position. Thus, since the ultrasonic vibration is continued with an increased amplitude, it is possible to finish the incision of living tissue within a shorter time than in the cases shown in FIGS. 8A and 8B.

The following can be stated for the treatment system 10 according to the present embodiment in light of the above descriptions.

The support section 100 provided on or near the inner circumferential surface of the distal end portion 42a of the sheath 42 is located ahead of, along the central axis C, a position corresponding to a node of the vibration of the probe 24 at a state when ultrasonic vibration is transmitted, and the separated position to the probe 24 is switched to the support position at which the support section 100 abuts the probe 24 for support when the probe 24 is bent in accordance with a load applied to the treatment section 74. The support section 100 is made of a metallic material having conductivity that moves in response to the vibration of the probe 24 in a state when ultrasonic vibration is transmitted when the probe 24 is bent and is switched from the separate position to the support position, and that absorbs the vibration transmitted to the probe 24 when the probe 24, in a state when ultrasonic vibration is transmitted, is switched from the separate position to the support position. Furthermore, it is possible to match (resonate with) the vibration of the probe 24 when the probe 24, in a state when ultrasonic vibration is transmitted, is abutted to the support section 100, thereby preventing damaging the probe 24. Thus, even if the probe 24 to which vibration is transmitted is bent and the probe 24 comes close to the sheath 42 and is abutted to the support section 100, it is possible to prevent breakage of the probe 24.

The support section 100 prevents abrasion of the probe 24, and preferably has abrasion resistance to prevent abrasion to the support section 100 itself by the probe 24. In other words, it is possible to prevent to the greatest extent possible abrasion of the support section 100 by the probe 24 to which ultrasonic vibration is transmitted. For this reason, even if the probe 24 to which vibration is transmitted is bent and the probe 24 comes close to the sheath 42 and is abutted to the support section 100, it is possible to prevent breakage of the support section 100 and the sheath 42 in which the support section 100 is arranged.

Since the support section 100 is formed as described above, even if the probe 24 comes close to the sheath 42 and is abutted to the support section 100, it is possible to continue resonating ultrasonic vibration to the probe to permit choosing to continue the treatment of living tissue. Accordingly, the treatment device 12 according to the present embodiment can reliably perform treatment for living tissue, such as incision, etc. in a state where the probe 24 is bent and the bend of the probe 24 is supported by the support section 100.

It should be noted that in the example shown in FIG. 8D, the controller 16 stops outputting ultrasonic vibration at the second stage when the probe 24 is supported by the support section 100. Thus, it is possible to reliably prevent transmission of ultrasonic vibration to the sheath 42 through the support section 100 and to extend the life of the probe 24 and the sheath 42.

According to the treatment system 10 of the present embodiment, the user can perform a treatment as appropriate in accordance with the user's preference; for example, the user can continue outputting ultrasonic vibration until the incision of living tissue is completed. For example, even if the probe 24, in a state when ultrasonic vibration is transmitted, is abutted to the support section 100, it is possible not to interfere with an incision treatment for living tissue, while maintaining outputting ultrasonic vibration as appropriate, and to stop ultrasonic vibration after completing the incision of living tissue.

As a material for the support section 100 according to the present embodiment, a vibration-dampening alloy, for example, is preferable. The support section 100, made of a vibration dampening alloy, can be moved together with the probe 24 in a state when ultrasonic vibration is transmitted, and can absorb the ultrasonic vibration transmitted to the probe 24 when the probe 24, in a state when ultrasonic vibration is transmitted, is bent at the support position with respect to the support section 100. The support section 100 dampens, in other words, attenuates vibration as soon as possible in accordance with its dampening capacity, compared to other metallic materials in the same shape, such as a stainless alloy, etc. Thus, using a material having a high dampening capacity and high matching properties as the support section 100 allows the support section 100 to exert a braking effect to the ultrasonic vibration of the probe 24 more quickly when vibration energy is transmitted from the probe 24 to the support section 100 made of a vibration-dampening alloy. In other words, the support section 100 made of the vibration-dampening alloy can function as a vibration-dampening section to dampen (attenuate) ultrasonic vibration of the probe 24.

Next, the first variation of the treatment system 10 of the first embodiment will be described with reference to FIG. 9.

In the first embodiment, as the support section 100, a metallic material, having conductivity that moves in response to the vibration of the probe 24 in a state when ultrasonic vibration is transmitted when the probe 24 is bent and is switched from the separate position to the support position, and that absorbs the vibration transmitted to the probe 24 when the probe 24 in a state of ultrasonic vibration is transmitted, is switched from the separate position to the support position.

As shown in FIG. 9, it is preferable to arrange, adjacent to the support section 100, a resin material 98 having electrical insulation properties such as a PTFE material, for example, and having heat and abrasion resistance. The resin material 98 is preferably in approximately an arc shape with a certain angle (approximately U-shaped), or in a ring shape, etc. The resin material 98 is preferably arranged on the more distal end side of the inner circumferential surface of the distal end portion 42a of the sheath 42 than the support section is, and is preferably formed so as to abut the probe 24 before the support section 100 abuts the probe 24 when the probe 24 is bent. In other words, the probe 24 is abutted to the resin material 98 first, and when the bent amount becomes significant when an external force is applied to the treatment section 74, etc., the resin material 98 is cut away by the probe 24, or the probe 24 is elastically deformed so as to be abutted to the support section 100. Thus, even when the probe 24 is bent in a state when ultrasonic vibration is or is not transmitted to the probe 24, it is possible to support the probe 24 with the resin material'98. The support section 100 should be formed with higher resistance for heat and abrasion than the resin material 98 is.

Since the other structures and working in the first variation are the same as those described in the first embodiment, the description thereof is omitted.

Next, the second variation of the treatment system 10 according to the first embodiment is described with reference to FIG. 10.

As shown in FIG. 10, the controller 16 has a first detection section 108a and a second detection section 108b, instead of the impedance detection section 108 (see FIG. 6). The power output section 106 is connected to the first detection section 108a, and a different power output section 107 is connected to the second detection section 108b.

The first detection section 108a is electrically coupled to the transducer 62. The power output section 106 supplies a current to the transducer 62, and can provide a potential difference between piezoelectric elements of the transducer 62. In other words, the first detection section 108a can detect acoustic impedance Z of the transducer Z by a current and a voltage applied to the transducer 62. Thus, the first detection section 108a can be used to detect a mechanical load (see FIG. 7A) explained in the first embodiment.

The second detection section 108b is electrically connected to the probe 24 and the support section 100. The other power output section 107 provides a potential difference between the probe 24 and the support section 100 to supply a potential difference. As a current flows through the living tissue between the probe 24 and the support section 100 at this time, the impedance between the probe 24 and the support section 100 can be detected. Thus, the second detection section 108b can be used to detect a threshold of an electric load between the probe 24 and the support section 100.

In FIG. 7B, an electric load between the probe 24 and the support section 100 (ease of the flow of electricity) is measured. The closer the probe 24 is to the support section 100, the more easily the electricity flows between the probe 24 and the support section 100 via fluids, etc. of the living tissue. In other words, the second detection section 108a can detect an impedance Z that varies depending on a state between the probe 24 and the support section 100. Thus, an electric load, i.e., impedance Z between the probe 24 and the support section 100 falls rapidly when the probe is abutted to the support section 100. If a threshold is empirically set for the acoustic impedance at this time, it is possible to recognize switching of the probe 24 from the separate position to the support position with respect to the support section 100.

For example, the memory 104 stores a threshold of a mechanical load (i.e., difficulty of movement of probe 24) applied to the probe 24 detected at the first detection section 108a (see FIG. 7A), and a threshold of an electric load (i.e., ease of the flow of electricity) between the probe 24 and the support section 100 detected at the second detection section 108b (see FIG. 7B), etc.

As described in the first embodiment, a control is switched from the first stage control to the second stage control when a treatment is performed. At this time, upon the impedance shown in FIG. 7B reaching a threshold in addition to the impedance Z shown in FIG. 7A reaching a threshold (the impedance decreases to reach the threshold), the stage switching section 112 of the controller 16 preferably switches the control from the first stage control to the second stage control. In other words, the stage switching section 112 of the controller 16 switches the control from the first stage control to the second stage control, using two thresholds as judgment data. In this case, the controller 16 can determine the switching from the separate position to the support position more reliably.

Since the other structures and working in the second variation are the same as those described in the first embodiment, the description thereof is omitted.

Next, the treatment system 10a according to the second embodiment is described with reference to FIGS. 11 to 12A. The present embodiment is a variation of the first embodiment including each of the variations; accordingly, the same members described in the first embodiment and the members having the same functions will be referenced with the same reference numbers, and detailed descriptions will be omitted.

An example of using high frequency energy as well as ultrasonic vibration energy in the treatment system 10a according to the present embodiment will be described. The treatment system 10a according to the present embodiment can apply high frequency energy and ultrasonic vibration energy to the probe 24 at the same time, as shown in FIG. 12A. Herein, an example of applying high frequency energy to living tissue will be explained as a bi-polar type of application; however, a mono-polar type is also preferable.

As shown in FIG. 11, a pin 130 which is electrically connected to the probe 24 is arranged in the operation main body 52 of the treatment unit 22 of the treatment device unit 12a of the treatment system 10a according to the present embodiment.

The probe 24, to which ultrasonic vibration is transmitted, also functions as an electrode (first electrode) for performing a treatment on living tissue with high frequency energy through the pin 130. As shown in FIG. 12A, the probe 24 is electrically connected to the high frequency energy output section 132 of the controller 16.

The pressing section 84 of the action portion 44 includes a pad 92 and an electrode (second electrode) 94 for performing a treatment on living tissue with high frequency energy. Specifically, the electrode 94 is embedded in the pad 92. The electrode 94 is electrically connected to the high frequency energy output section 132 of the controller 16, and to the support section 100.

Thus, when living tissue is pressed down by the pad 92 of the pressing section 84 of the action portion 44 against the treatment section 74 of the probe 24, high frequency energy can be applied by the high frequency energy output section 132 to the living tissue between the probe (first electrode) 24 and the electrode (second electrode) 94 embedded in the pad 92. Thus, it is possible to provide hemostasis to living tissue with high frequency energy, while incising the living tissue with energy of the ultrasonic vibration transmitted to the probe 24.

Herein, since the support section 100 and the electrode (second electrode) 94 embedded in the pad 92 are electrically connected, a short circuit occurs between the probe 24 and the electrode (second electrode) 94 embedded in the pad 92 when the probe 24 is switched from the separate position to the support position at which the probe 24 is supported by being abutted to the support section 100, while high frequency energy is applied between the probe 24 and the electrode (second electrode) 94 embedded in the pad 92. Thus, the short circuit can be detected at the anomaly detection section 110 of the controller 16 to stop outputting high frequency energy from the high frequency energy output section 132. Furthermore, the output of ultrasonic vibration energy can be continuously shifted from the first stage to the second stage by the signals from the high frequency energy output section 132.

Since the other structures and working in the second embodiment are the same as those described in the first embodiment, the description thereof is omitted. Thus, in the treatment system 10a according to the present embodiment, the mechanical load of the probe 24 and the electric load between the probe 24 and the support section 100 are preferably detectable, and a control can be performed from the first stage to the second stage as appropriate to perform a treatment in accordance with a user's preference.

Next, a variation example of the treatment system 10a according to the second embodiment is described with reference to FIG. 12B.

As shown in FIG. 12B, the controller 16 has a short circuit detection circuit (detection section) 142.

The probe 24 receives ultrasonic vibration and functions as an electrode (a first electrode). The probe 24 is electrically connected to the high frequency energy output section 132 of the controller 16. The probe 24 is further electrically connected to the short circuit detection circuit 142.

The pressing section 84 of the action portion 44 has a pad 92 and an electrode (a second electrode) 94. Specifically, the electrode 94 is embedded in the pad 92. The electrode 94 is electrically connected to the high frequency energy output section 132 of the controller 16.

It should be noted that the support section 100 is electrically connected to the short circuit detection circuit 142, but not to the electrode 94 of the action portion 44.

When the probe 24 is switched from the separate position to the support position at which the probe 24 is supported by being abutted to the support section 100, a short circuit occurs between the probe 24 and the support section 100, and the short circuit can be detected by the short circuit detection circuit 142. At this time, signals are sent from the short circuit detection circuit 142 to the CPU 102 to stop outputting high-frequency energy from the high frequency energy output section 132.

In the treatment system 10a according to this variation, a short circuit does not occur between the probe (first electrode) 24 and the electrode (second electrode) 94 of the action portion 44. Thus, when switching from the separate position to the support position, the controller 16 can continue outputting ultrasonic vibration to the probe 24, and continue outputting high-frequency energy. In other words, even after switching from the separate position to the support position, it is possible to apply high frequency energy between the probe 24 and the electrode 94 of the action portion 44 while transmitting ultrasonic vibration to the probe 24.

The treatment system 10a according to this variation can use a detection of a short circuit between the probe 24 and the support section 100 by the short circuit detection circuit 142 as a trigger for switching the ultrasonic vibration control from the first stage to the second stage.

Next, the third embodiment is described with reference to FIG. 13A. The present embodiment is a variation of the first and second embodiments including each of their variations; accordingly, the same members described in the first embodiment and the members having the same functions will be referenced with the same reference numbers, and detailed descriptions will be omitted.

In the treatment device 12 according to the present embodiment, the support section 100 shown in FIGS. 3A to 5B is not always necessary, and a probe cover (extended portion) 150 is arranged as a support section on the distal end side with respect to the distal end of the sheath 42, as shown in FIG. 13A. The probe cover 150 is preferably made of a material that is the same as the material of the support section 100 described in the first embodiment. In other words, the probe cover 150 is preferably made of a vibration-dampening alloy, for example. Thus, when an external force is applied to the probe 24 in a state when ultrasonic vibration is transmitted, and the treatment section 74 of the probe 24 is bent and abutted to the probe cover 150, the probe cover 150 absorbs the vibration, as described in the first embodiment. Accordingly, it is possible to prevent damaging the probe 24, such as getting scratches on the probe 24, if the treatment section 74 of the probe 24 is abutted to the probe cover 150. The breakage of the probe cover 150 can, of course, be prevented. The outer circumferential surface of the probe cover 150 should be coated with a material having heat resistance and electrical insulation properties, like PTFE. Thus, it is possible to prevent affecting tissue in the vicinity of treatment target living tissue by an ultrasonic treatment or a high frequency treatment by coating the probe cover 150 with a material having electrical insulation properties.

The jaw 82 and the pressing section 84 of the action portion 44 of the treatment device 12 according to the present embodiment are supported by the pivotal portion 86. Thus, the pressing section 84 according to the present embodiment is formed as a so-called see-saw jaw that rotatably moves about the jaw 82 like a see-saw. The action portion 44 described in the first and second embodiments is also preferably formed as a see-saw jaw.

Since the other structures and working in the third embodiment are the same as those described in the first and second embodiments, the description thereof is omitted. Thus, in the treatment system 10 according to the present embodiment, the mechanical load of the probe 24 and the electric load between the probe 24 and the support section 100 are detectable, and a control can be performed from the first stage to the second stage as appropriate to perform a treatment in accordance with a user's preference.

Next, the first variation of the treatment system 10 according to the third embodiment is described with reference to FIGS. 13B and 13C.

As shown in FIG. 13B, a probe cover (extended portion) 160 in this variation is made of a resin material having electrical insulation properties, such as PTFE. A support section 162 made of a same material as the material of the support section 100 described in the first embodiment is preferably arranged in the probe cover 160. The support section 162 is arranged in closer proximity to the treatment section 74 of the probe 24 than the inner circumferential surface of the probe cover 160.

Only one support section 162 may be arranged in the probe cover 160 as shown in FIG. 13B, and a plurality of support sections 162 may be arranged discretely in the probe cover 160 as shown in FIG. 13C. If there are a plurality of support sections 162, the height of each of the support sections 162 may be the same or different.

In the treatment system 10 according to this variation, the mechanical load of the probe 24 and the electric load between the probe 24 and the support section 162 are detectable, and a control can be performed from the first stage to the second stage as appropriate to perform a treatment in accordance with a user's preference.

Next, the second variation of the third embodiment is described with reference to FIGS. 14A and 14B. Note that the action portion 44 is omitted in FIGS. 14A and 14B.

As shown in FIGS. 14A and 14B, the sheath 42 has an extended portion 170 as a support section which is extended on the distal side of the distal end of the sheath 42. The extended portion 170 is a plane, and is made of a same material as the material of the support section 100 described in the first embodiment. In this variation, unlike the probe cover 150 shown in FIG. 13A, the width of the extended portion 170 is the same as or smaller than the diameter of the probe 24, specifically, the diameter of the treatment section 74 of the probe 24.

Even though the extended portion 170 as a support section is formed in such a manner, when (the treatment section 74 of) the probe 24, in a state when ultrasonic vibration is transmitted, is abutted to the extended portion 170, the extended portion 170 can function like the support section 100 described in the first and second embodiments.

Thus, most of the probe 24, which is opposed to the action portion 44, may be covered (see FIGS. 13A to 13C); even if not covered, the probe 24 may be formed so as to be supported by the extended portion 170 being abutted to the extended portion 170.

Furthermore, in the treatment system 10 according to this variation, the mechanical load of the probe 24 and the electric load between the probe 24 and the support section 170 as a support section are detectable, and a control can be performed from the first stage to the second stage as appropriate to perform a treatment in accordance with a user's preference.

Some embodiments have been specifically described with reference to the drawings; the invention is not limited to the above-described embodiments and includes all embodiments that can be carried out within the scope not deviating from the substance of the above-described embodiments.

### Reference Signs List

10 ... Treatment System, 12 ... Treatment Device, 14 ... Ultrasonic Transducer Unit, 16 ... Controller, 22 ... Treatment Unit, 24 ... Probe, 32 ... Insertion Section, 42 ... Sheath (Cylindrical body), 42a ... Sheath Distal End, 44 ... Action Section, 46 ... Pivotal Shaft, 72 ... Vibration Transmitting Section, 74 ... Treatment Section, 76 ... Holding Member, 82 ... Jaw, 84 ... Pressing Section, 92 ... Pad (Shock Absorbing Section), 96 ... Gripping Surface, 100 ... Support Section (Probe Retainer).

## Claims

1. A treatment device comprising:
a cylindrical body having a central axis;
a probe having conductivity in which ultrasonic vibration is transmitted from a proximal end thereof to a distal end thereof and which is inserted into the cylindrical body and extends toward a distal end side with respect to a distal end of the cylindrical body; and
a support section having conductivity which is separated forward or backward, along the central axis, of a position corresponding to a node of vibration of the probe in a state when the ultrasonic vibration is transmitted, for which a separate position separated from the probe is switched to a support position at which the support section abuts the probe to support the probe when bent in a direction off of the central axis in accordance with a load which is applied to the probe, and the support section which is subserviently moved with the vibration of the probe and absorbs the vibration transmitted in the probe when the support section is at the support position.

2. The treatment device according to claim 1, wherein the support section is arranged on an inner circumferential surface of the cylindrical body and on the distal end portion or a part adjacent to the distal end portion of the cylindrical body.

3. The treatment device according to claim 1, wherein the support section is arranged on a distal end side with respect to the distal end of the cylindrical body.

4. The treatment device according to claim 1, wherein the support section includes an action portion which is configured to apply a load to the probe in a direction off of the central axis.

5. The treatment device according to claim 4, wherein the support section is arranged opposite to the action portion with respect to the probe.

6. The treatment device according to claim 1, wherein the support section is made of a vibration dampening alloy which is subserviently moved with the probe to which the ultrasonic vibration is transmitted and which is configured to absorb the ultrasonic vibration transmitted to the probe when the probe which transmits the ultrasonic vibration is bent and is at the support position with respect to the support section

7. The treatment device according to claim 1, wherein the support section has abrasion resistance to prevent from grinding the probe and to prevent from being grinded by the probe.

8. A treatment system comprising:
the treatment device recited in claim 1; and
a controller having a detection section which is configured to detect a switching when the probe to which the ultrasonic vibration is transmitted is abutted to the support section and the switching is switched from a separate position to a support position.

9. The treatment system according to claim 8, wherein the detection section is configured to detect a short circuit between the probe and the support section.

10. The treatment system according to claim 8,
wherein the detection section is configured to detect impedance which varies in accordance with a state between the probe and the support section.

11. The treatment system according to claim 8,
wherein the detection section is configured to detect a mechanical load to the probe.

12. The treatment system according to claim 8, further comprising an ultrasonic transducer arranged at the proximal end of the probe and is configured to oscillate ultrasonic vibration from the proximal end of the probe to the distal end when power is supplied from the controller,
wherein the controller is configured to supply power to the ultrasonic transducer to maintain amplitude of the ultrasonic vibration to the ultrasonic transducer when the switching is detected at the detection section.

13. The treatment system according to claim 9, further comprising an ultrasonic transducer arranged at the proximal end of the probe and is configured to oscillate ultrasonic vibration from the proximal end of the probe to the distal end when power is supplied from the controller,
wherein the controller supplies power intermittently to the ultrasonic transducer based on a signal from the detection section when the short circuit is detected at the detection section.

14. The treatment system according to claim 9, further comprising an ultrasonic transducer arranged at the proximal end of the probe and is configured to oscillate ultrasonic vibration from the proximal end of the probe to the distal end when power is supplied from the controller,
wherein the controller stops supplying power to the ultrasonic transducer based on a signal from the detection section when the short circuit is detected at the detection section.

15. The treatment system according to claim 8, further comprising an ultrasonic transducer arranged at the proximal end of the probe and is configured to oscillate ultrasonic vibration from the proximal end of the probe to the distal end when power is supplied from the controller,
wherein the controller is configured to supply power to the ultrasonic transducer to reduce an amplitude of the ultrasonic transducer compared to an amplitude immediately before the switching is detected at the detection section when the switching is detected at the detection section.

16. The treatment system according to claim 8, further comprising an ultrasonic transducer arranged at the proximal end of the probe and is configured to oscillate ultrasonic vibration from the proximal end of the probe to the distal end when power is supplied from the controller,
wherein the controller is configured to supply power to the ultrasonic transducer to increase an amplitude of the ultrasonic transducer compared to an amplitude immediately before the switching is detected at the detection section when the switching is detected at the detection section.

17. The treatment system according to claim 8,
wherein the controller includes an alarm generating section which generates an alarm sound when the switching is detected.
